(19) Europäisches Patentamt
European Patent Office
Office européen des brevets

(11) Veröffentlichungsnummer: **0 005 806**
**B1**

(12) # EUROPÄISCHE PATENTSCHRIFT

(45) Veröffentlichungstag der Patentschrift: **28.07.82**

(51) Int. Cl.³: **A 01 N 37/20** // C07C103/50

(21) Anmeldenummer: **79101607.4**

(22) Anmeldetag: **25.05.79**

(54) **Verwendung von Aminohydroxystearinsäureamiden.**

(30) Priorität: **31.05.78 DE 2823685**

(43) Veröffentlichungstag der Anmeldung: **12.12.79 Patentblatt 79/25**

(45) Bekanntmachung des Hinweises auf die Patenterteilung: **28.07.82 Patentblatt 82/30**

(84) Benannte Vertragsstaaten: **FR GB**

(56) Entgegenhaltungen:
**DE-B-1 174 018**
**FR-A-2 249 871**

(73) Patentinhaber: **Henkel Kommanditgesellschaft auf Aktien, Postfach 1100 Henkelstrasse 67, D-4000 Düsseldorf 1 (DE)**

(72) Erfinder: **Tesmann, Holger, Dr., Heinrich-Hertz-Strasse 16, D-4000 Düsseldorf-13 (DE)**
Erfinder: **Rutzen, Horst, Dr., Falkenweg 12, D-4018 Langenfeld (DE)**
Erfinder: **Börner, geb. Schlenzka, Elke, Meliesallee 32, 4000 Düsseldorf 13 (DE)**

EP 0 005 806 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

ACTORUM AG

# Verwendung von Aminohydroxystearinsäureamiden

Die Erfindung betrifft die Verwendung von Aminohydroxystearinsäureamiden, in denen Amino- und Hydroxygruppierungen in vicinaler Stellung an der Alkylkette angeordnet sind, als antimikrobielle Wirkstoffe.

Es wurde gefunden, dass Aminohydroxystearinsäureamide der Formel I

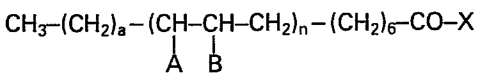

I

in der die Kombinationen n=1 und a=7, oder n=2 und a=4, oder n=3 und a=1 gelten und von den Substituenten A und B jeweils einer eine Hydroxylgruppe bedeutet, während der verbleibende Substituent B oder A und der Substituent X einen Rest der Formel II

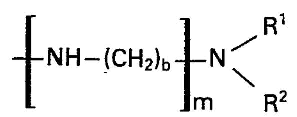

II

darstellt, in der b ganze Zahlen von 2 bis 6, m die Zahlen 1 oder 2, $R^1$ und $R^2$ unabhängig voneinander Wasserstoff oder einen Alkylrest mit 1 bis 12 Kohlenstoffatomen bedeuten, sich mit ausgezeichneter Wirkung als antimikrobielle Wirkstoffe verwenden lassen.

Die Verbindungen der Formel I werden zweckmässigerweise durch Umsetzen von Epoxystearinsäureestern der Formel III

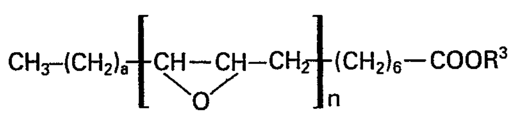

III

mit Di- und Triaminen der Formel IV

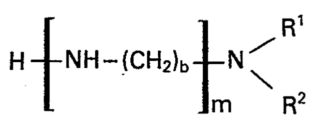

IV

hergestellt. Dabei haben a, b, m, n, $R^1$ und $R^2$ dieselbe Bedeutung wie in den Formeln I und II. $R^3$ steht für einen Alkylrest mit 1 bis 5 Kohlenstoffatomen oder den Rest eines mehrwertigen Alkohols mit 2 bis 6 Kohlenstoffatomen, dessen übrige Hydroxylgruppen ebenfalls mit Epoxyfettsäureestern, gegebenenfalls auch mit gesättigten und ungesättigten Fettsäureestern verestert sind. Die Epoxystearinsäureester der Formel III werden mit wenigstens n+1 Mol eines Amins der Formel IV gegebenenfalls in Gegenwart eines polaren Lösungsmittels bei Temperaturen von 130 bis 200° C umgesetzt. Flüchtige Nebenprodukte werden nach beendeter Reaktion abgetrennt.

Werden die Epoxystearinsäureester der Formel III mit den Aminen der Formel IV in einem Lösungsmittel miteinander umgesetzt, so sind hierfür besonders die niederen Alkohole wie Methanol, Äthanol, Isopropanol, aber auch Äthylenglykol oder Glycerin geeignet. Diese Alkohole können auch mit Wasser gemischt werden. Auch ein Zusatz katalytischer Mengen von Alkalien wie z.B. Natriumhydroxid ist zweckmässig.

In den Epoxystearinsäueestern der Formel III besteht die Säurekomponente aus epoxidierter Ölsäure, Linolsäure oder Linolensäure, d.h. aus epoxidierten Fettsäuren der Kettenlänge $C_{18}$. Die genannten Säuren enthalten je nach ihrer Herkunft auch Anteile von ungesättigten $C_{14}$-, $C_{16}$-, $C_{20}$- oder $C_{22}$-Fettsäuren in untergeordneten Mengen von insgesamt bis zu 25 Gew.-%. Wegen ihrer guten Zugänglichkeit sind die Epoxyester der Formel III, die sich von der in zahlreichen natürlichen Fetten vorkommenden Ölsäure ableiten, als Ausgangsmaterial für die Herstellung der erfindungsgemäss zu verwendenden Verbindungen von besonderem praktischem Interesse.

Die Alkoholkomponente der Verbindungen der Formel III besteht aus niederen aliphatischen Alkoholen wie Methanol, Äthanol, Propanol, Isopropanol, n-Butanol und n-Pentanol oder aus aliphatischen Di- und Polyolen wie z.B. Äthylenglykol, Propylenglykol, Hexandiol-(1,6) und Glycerin.

Als Ester der Formel III werden die epoxidierten Fettsäureester von einwertigen Alkoholen mit 1 bis 5 Kohlenstoffatomen, insbesondere die Methylester, bevorzugt zur Herstellung der erfindungsgemäss zu verwendenden Verbindungen eingesetzt.

Geht man bei der Herstellung der erfindungsgemäss zu verwendenden Verbindungen der Formel I von epoxidierten Estern mehrwertiger Alkohole aus, insbesondere von Triglyceriden, wie sie aus natürlichen Fettsäurequellen zugänglich sind, dann können im Reaktionsprodukt auch noch Glycerin oder Fettsäureteilglyceride vorhanden sein, wodurch die vorteilhafte Wirkung der Verfahrensprodukte praktisch nicht beeinträchtigt wird. Bei diesen als Nebenprodukten anfallenden Fettsäurederivaten von gesättigten oder ungesättigten Fettsäuren handelt es sich um solche, die im allgemeinen 10 bis 22, insbesondere 12 bis 18 Kohlenstoffatome enthalten.

Es ist demnach nicht erforderlich, die den Epoxyverbindungen der Formel III zugrunde liegenden reinen Ölsäure-, Linolsäure- und Linolensäureester einzusetzen; vielmehr können als Ausgangsstoffe epoxierte Fettsäureestergemische eingesetzt werden, wie sie aus den natürlichen Pflanzen- und Tierfetten, wie z.B. Olivenöl, Sojaöl, Rüböl, Baumwollsaatöl, Leinöl, Talg, Fischölen, Tallöl usw. anfallen.

Bei den Aminen der Formel IV handelt es sich um aliphatische Diamine und Triamine, wir z.B. Äthylendiamin, Trimethylendiamin, Tetramethylendiamin, Pentamethylendiamin, Hexamethylendiamin, Di-äthylentriamin und Di-trimethylentriamin sowie um deren unsymmetrisch substituierte Mono- und Dialkylderivate wie z.B. N,N-Dimethylaminoäthylenamin, N,N-Dimethylaminopropylamin oder N-Dodecylaminopropylamin.

Zur Herstellung von erfindungsgemäss zu verwendenden Verbindungen wird bevorzugt die Umsetzung eines Epoxystearinsäureesters eines einwertigen $C_1$-$C_3$-Alkohols mit 2 bis 10 Mol Amin der Formel IV herangezogen. Die Umsetzung wird in der Regel in einem polaren Lösungsmittel aus der Gruppe der einwertigen, zweiwertigen und dreiwertigen Alkohole mit 1 bis 3 Kohlenstoffatomen bei einer Temperatur von 130 bis 160° C, gegebenenfalls in Gegenwart einer katalytischen Menge Alkali durchgeführt. Die Reaktionsdauer beträgt im allgemeinen 2 bis 12 Stunden. Die Reaktion gilt als beendet, wenn sich praktisch keine Epoxygruppen mehr nachweisen lassen. Bei der Aufarbeitung des Reaktionsgemisches wird das Produkt von den flüchtigen Bestandteilen, d.h. vom Lösungsmittel und von dem im Überschuss eingesetzten Amin der Formel IV abgetrennt, was im allgemeinen durch Destillation oder durch Auswaschen mit einer gesättigten wässrigen Salzlösung, beispielsweise mit Kochsalz-, Soda oder Natriumsulfatlösung, geschieht.

Bei den erfindungsgemäss zu verwendenden Produkten handelt es sich beispielsweise um das Umsetzungsprodukt von Epoxystearinsäuremethylester mit Äthylendiamin, d.h. um ein Gemisch der N-(2-Aminoäthyl)-amide der 9-(2-Aminoäthyl)-10-hydroxystearinsäure und der 10-(2-Aminoäthyl)-9-hydroxystearinsäure, ferner um die entsprechenden, durch Umsetzung dieses Epoxyesters mit Trimethylendiamin, Tetramethylendiamin, Pentamethylendiamin, Hexamethylendiamin, Di-äthylentriamin, N-Methyläthylendiamin, N,N-Dimethylaminopropylamin und N-Dodecylaminopropylamin hergestellten Aminohydroxystearinsäureamide. Weiterhin sind zu nennen: Das Umsetzungsprodukt aus epoxydiertem Olivenölfettsäuremethylester und Trimethylendiamin; das Umsetzungsprodukt aus dem epoxidierten Methylester der Tallölfettsäuren mit N,N-Dimethyläthylendiamin; das Umsetzungsprodukt aus dem Gemisch von 50 Gew.-% epoxydiertem Talgölfettsäuremethylester und 50 Gew.-% epoxydiertem Sojafettsäuremethylester mit Hexamethylendiamin; das Umsetzungsprodukt aus dem Gemisch von 70 Gew.-% epoxydiertem Sojafettsäuremethylester und 30 Gew.-% epoxydiertem Fischölsäuremethylester mit Di-trimethylentriamin und das Umsetzungsprodukt aus epoxydiertem Olivenöl mit Äthylendiamin.

Zur Verwendung in antimikrobiellen Mitteln können die Aminohydroxystearinsäureamide in flüssige, pastenförmige oder feste Zubereitungen eingearbeitet werden, z.B. in wässrige Lösungen, Suspensionen, Emulsionen und Lösungen in organischen Lösungsmitteln. Derartige antimikrobielle Mittel können auf den verschiedensten Gebieten zum Einsatz gelangen, z.B. als Reinigungs-, Desinfektions- und Konservierungsmittel für Textilien, Fussböden, Krankenhauseinrichtungen und -instrumente, gewerbliche Betriebe, wie Molkereien, Brauereien und Wäschereien. In antimikrobiellen Mitteln werden die erfindungsgemäss zu verwendenden Produkte in Mengen von 0,1 bis 10 Gew.-%, vorzugsweise 0,5 bis 5 Gew.-%, bezogen auf das gesamte antimikrobielle Mittel, eingesetzt.

Ausserdem können die Aminohydroxystearinsäureamide zur Konservierung von technischen Produkten, die dem Befall durch Bakterien und Pilze oder sonstiger mikrobieller Zerstörung unterliegen, wie beispielsweise Stärkekleistern, Keimen, Dispersionsfarben sowie Schneid- und Bohrölen, dienen. Für diesen Verwendungszweck ist im allgemeinen ein Zusatz von 0,1 bis 2 Gew.-%, bezogen auf das zu konservierende Material, ausreichend.

Die nachfolgenden Beispiele sollen den Gegenstand der Erfindung näher erläutern, ohne ihn hierauf zu beschränken.

Beispiele

I. Herstellung der erfindungsgemäss zu verwendenden Aminohydroxystearinsäureamide

Beispiel 1

Umsetzungsprodukt aus Epoxystearinsäuremethylester und Äthylendiamin (Substanz A).

Eine Mischung aus 66,8g (0,21 Mol) Epoxystearinsäuremethylester (4,78 Gew.-% Epoxydsauerstoff), 60,1g (0,77 Mol) Äthylendiamin, 200ml Methanol und 80ml Wasser wurde im Autoklaven auf 130° C erhitzt und 12 Stunden lang bei dieser Temperatur gehalten. Nach dem Erkalten wurde die methanolische Lösung filtriert, das Filtrat eingeengt und überschüssiges Äthylendiamin unter vermindertem Druck abdestilliert. Als Reaktionsprodukt wurde eine bräunliche viskose Masse erhalten. Brechungsindex $n_D^{25}$ 1,3720 (25 gew.-%ige Lösung in Methanol).

Beispiel 2

Umsetzungsprodukt aus Epoxystearinsäuremethylester und Hexamethylendiamin (Substanz B).

78g (0,25 Mol) Epoxystearinsäuremethylester (4,57 Gew.–% Epoxidsauerstoff), 73g (0,63 Mol) Hexamethylendiamin und 120ml Methanol wurden im Autoklaven auf 200° C erhitzt und 8 Stunden lang bei dieser Temperatur gehalten. Nach dem Erkalten wurde filtriert. Aus dem Filtrat wurde das Lösungsmittel und überschüssiges Hexamethylendiamin unter vermindertem Druck abdestilliert. Als Reaktionsprodukt blieb eine gelborange zähflüssige Masse zurück.

Brechungsindex $n_D^{25}$ 1,3705 (25 gew.-%ige Lösung in Methanol).

Beispiel 3

Umsetzungsprodukt aus Epoxystearinsäuremethylester und Di-trimethylendiamin (Substanz C).

62,5g (0,2 Mol) Epoxystearinsäuremethylester (4,57 Gew.-% Epoxidsauerstoff). 65g (0,5 Mol) Ditrimethylentriamin und 100ml Methanol wurden im Autoklaven 6 Stunden lang auf 160° C erhitzt. Nach dem Filtrieren und Eindampfen des Filtrates

unter vermindertem Druck wurde das Reaktionsprodukt als bräunliche Paste erhalten.

Brechungsindex $n_D^{25}$ 1,3760 (25 gew.-%ige Lösung in Methanol).

Beispiel 4

Umsetzungsprodukt aus Epoxystearinsäuremethylester und N,N-Dimethylpropylendiamin (Substanz D).

Ein Gemisch aus 147,5g (1,45 Mol) N,N-Dimethylpropylendiamin und 32,5g Glycerin wurde auf 130° C erhitzt und unter Rühren 85g (0,27 Mol) Epoxystearinsäuremethylester (4,41 Gew.-% Epoxidsauerstoff) unter Rühren zugetropft. Das Gemisch wurde noch 3 Stunden lang bei 130° C gerührt. Danach wurden die flüchtigen Bestandteile bei einer Badtemperatur von 160–185° C im Ölpumpenvakuum (0,01 Torr) abdestilliert. Als Reaktionsprodukt wurde eine gelb-braune Paste erhalten.

Brechungsindex $n_D^{25}$ 1,3652 (25 gew.-%ige Lösung in Methanol).

Beispiel 5

Umsetzungsprodukt aus Epoxystearinsäuremethylester und N-Dodecylpropylendiamin (Substanz E).

133g (0,43 Mol) Epoxystearinsäuremethylester (4,57 Gew.-% Epoxidsauerstoff), 230g (0,95 Mol) N-Dodecyltrimethylendiamin und 700ml Methanol wurden im Autoklaven auf 180° C erhitzt und 8 Stunden lang bei dieser Temperatur gehalten. Nach dem Erkalten wurde filtriert. Aus dem Filtrat wurden die flüchtigen Anteile unter vermindertem Druck abdestilliert. Als Reaktionsprodukt blieb eine bräunliche Paste zurück.

Brechungsindex $n_D^{25}$ 1,3658 (25 gew.-%ige Lösung in Methanol).

II. Antimikrobielle Wirksamkeit der erfindungsgemäss zu verwendenden Aminohydroxystearinsäureamide

Beispiel 6

Die mikrobistatische Wirksamkeit der Substanzen A bis E aus den Beispielen 1 bis 5 wurde gegenüber folgenden Testkeimsuspensionen bestimmt:

| | |
|---|---|
| 1) Staphylococcus aureus | $5 \times 10^7$ Keime/ml |
| 2) Escherichia coli | $5 \times 10^7$ Keime/ml |
| 3) Pseudomonas aeroginosa | $5 \times 10^7$ Keime/ml |
| 4) Candida albicans | $5 \times 10^7$ Keime/ml |

Die Hemmkonzentrationen der zu untersuchenden Produkte wurden mit Hilfe des Verdünnungstests nach den Richtlinien für die Prüfung chemischer Desinfektionsmittel der deutschen Gesellschaft für Hygiene und Mikrobiologie (1959) ermittelt. Die Versuche wurden in sterilen Reagenzröhrchen ausgeführt, die Standart-I-Bouillon (Merck) oder Bierwürze (8° BG) enthielten. Nach Zugabe der Wirkstoffe betrug das Nährlösungsvolumen in den Röhrchen jeweils 10ml. Anschliessend wurde jeweils 0,1ml Testkeimsuspension der angegebenen Konzentration in die Röhrchen gebracht. Die mit Bakterien geimpften Nährlösungsproben wurden 3 Tage lang bei 37° C im Brutschrank aufbewahrt. Die mit Candida albicans geimpften Proben wurden 3 Tage lang bei 30° C bebrütet. Danach wurde festgestellt, welche dem Nährmedium zugefügte Wirkstoffkonzentration das Wachstum der Keime gerade noch gehemmt hatte. Der auf diese Weise gefundene Wert wurde als Hemmkonzentration bezeichnet. Folgende Wirkstoffkonzentrationen in ppm wurden getestet:

1 000, 750, 500, 250, 100, 50, 10.

Für die Produkte A bis E wurden die in der nachstehenden Tabelle I aufgeführten Hemmkonzentrationen gegenüber den genannten Testkeimen gefunden.

Tabelle I

Hemmkonzentrationen der Produkte A bis E in ppm

| Produkt | Hemmkonzentration in ppm | | | |
|---|---|---|---|---|
| | Testkeim 1 | 2 | 3 | 4 |
| A | >10 | 50 | 50 | 50 |
| B | >10 | 10 | 50 | 10 |
| C | >10 | 50 | 50 | 50 |
| D | 50 | 100 | 250 | 250 |
| E | >10 | 50 | 250 | 100 |

Beispiel 7

Die mikrobizide Wirkung der Substanzen A bis E aus den Beispielen 1 bis 5 wurde gegenüber folgenden Testkeimsuspensionen bestimmt:

| | |
|---|---|
| 1) Staphylococcus aureus | $2 \times 10^8$ Keime/ml |
| 2) Escherichia coli | $3 \times 10^8$ Keime/ml |
| 3) Pseudomonas aeruginosa | $2 \times 10^8$ Keime/ml |
| 4) Candida albicans | $8 \times 10^7$ Keime/ml |

Die Abtötungszeiten der zu untersuchenden Produkte wurden mit Hilfe des Suspensionstests nach den Richtlinien für die Prüfung chemischer Desinfektionsmittel der deutschen Gesellschaft für Hygiene und Mikrobiologie (1959) ermittelt.

Die zu prüfenden Substanzen wurden zunächst in wenig Alkohol gelöst. Aus den äthanolischen Lösungen wurden durch Verdünnen mit entionisiertem Wasser Testlösungen hergestellt, die 500ppm, 250ppm und 100ppm Wirkstoff und maximal 1 Gew.-% Äthanol enthielten.

Den Richtlinien entsprechend wurden bei Raumtemperatur jeweils 0,1ml Testkeimsuspension in Reagenzgläser pipettiert. Hierzu wurden jeweils 10ml der oben beschriebenen Testlösungen gegeben. Nach Einwirkungszeiten von 2½, 5, 10, 20, 40, 60 und 120 Minuten wurde den Reagenzgläsern mit Hilfe einer Öse ein Tropfen Material entnommen und in 10ml Nährlösung, die 3% Tween 80 und 0,3% Lecithin als Enthemmer enthielt, überimpft. Das Nährmedium bestand bei den Bakterien aus 1 gew.-%iger Standard-I-Bouillon (Merck) bei Candida albicans aus 1 gew.-%iger Bierwürzelösung. Die mit Bakterien beimpften Proben wurden bei 37° C, die mit Candida albicans beimpften Proben bei 30° C bebrütet. Nach frühestens 5 Tagen wurden die Kulturen makroskopisch auf Wachstum beurteilt und auf diesem Weg die Abtötungszeiten ermittelt, die in der nachstehenden Tabelle II wiedergegeben sind.

Tabelle 2

Abtötungszeiten der Produkte A bis E bei Konzentrationen von 500 ppm, 250 ppm und 100 ppm in Minuten

| Substanz | ppm | Abtötungszeit in Minuten | | | |
|---|---|---|---|---|---|
| | | Testkeim | | | |
| | | 1 | 2 | 3 | 4 |
| A | 500 | 2,5 | 5 | 5 | 5 |
| | 250 | 2,5 | 5 | 5 | 10 |
| | 100 | 2,5 | 20 | 10 | 10 |
| B | 500 | 2,5 | 5 | 2,5 | 5 |
| | 250 | 2,5 | 5 | 2,5 | 5 |
| | 100 | 2,5 | 60 | 2,5 | 10 |
| C | 500 | 2,5 | 5 | 2,5 | 5 |
| | 250 | 2,5 | 5 | 2,5 | 10 |
| | 100 | 2,5 | 10 | 2,5 | 20 |
| D | 500 | 2,5 | 10 | 10 | 2,5 |
| | 250 | 5 | 10 | 10 | 2,5 |
| | 100 | 10 | 40 | 10 | 2,5 |
| E | 500 | 10 | 20 | 120 | 2,5 |
| | 250 | 20 | 40 | 120 | 5 |
| | 100 | 40 | 40 | >120 | 40 |

III. Verwendung als antimikrobielle Mittel

Nachstehend werden einige Beispiele für die Verwendung der erfindungsgemäss zu verwendenden Aminohydroxystearinsäureamide als antimikrobielle Mittel angegeben.

1. Desinfizierende Handwaschpaste

52 Gew.-T. Natriumlaurylsulfat (ca. 35% Waschaktivsubstanz)
3 Gew.-T. Kokosfettsäuremonoäthanolamid
43 Gew.-T. Bimsstein, fein gemahlen
2 Gew.-T. Substanz A

An Stelle der Substanz A kann mit gleich gutem Erfolg das Produkt C eingesetzt werden.

2. Schaumbad

70 Gew.-T. Natriumlauryläthersulfat (27–28 Gew.-% Waschaktivsubstanz)
5 Gew.-T. Kokosfettsäurediäthanolamid
0,5 Gew.-T. Substanz B
24,5 Gew.-T. Wasser

An Stelle der Substanz B können auch die Verbindungen A und C in die Zusammensetzung eingearbeitet werden.

3. Deodorant-Spray

10 Gew.-T. Octyldodecanol
1 Gew.-T. Parfüm
2 Gew.-T. Substanz D
87 Gew.-T. Äthanol
100 Gew.-T. Treibgas

In dieser Zusammensetzung kann die Substanz D durch das Produkt E ersetzt werden.

**Patentansprüche**

1. Verwendung von Aminohydroxystearinsäureamiden der Formel I

$$CH_3-(CH_2)_a-(\underset{A}{CH}-\underset{B}{CH}-CH_2)_n-(CH_2)_6-CO-X \qquad I$$

in der die Kombinationen n=1 und a=7, oder n=2 und a=4, oder n=3 und a=1 gelten und von den Substituenten A und B jeweils einer eine Hydroxylgruppe bedeutet, während der verbleibende Substituent B oder A und der Substituent X einen Rest der Formel II darstellt,

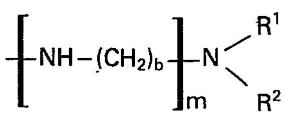
II

in der b ganze Zahlen von 2 bis 6, m 1 oder 2, $R^1$ und $R^2$ unabhängig voneinander Wasserstoff oder einen Alkylrest mit 1 bis 12 Kohlenstoffatomen bedeuten, als antimikrobielle Wirkstoffe, mit Ausnahme therapeutischer Verwendungen.

2. Verwendungen von Aminohydroxystearinsäureamiden nach Anspruch 1 zur Herstellung antimikrobieller Mittel in einer Menge von 0,1 bis 10 Gew.-%, vorzugsweise 0,5 bis 5 Gew.-%, bezogen auf das gesamte antimikrobielle Mittel.

3. Verwendung von Aminohydroxystearinsäureamiden nach Anspruch 1 zur Konservierung in einer Menge von 0,1 bis 2 Gew.-%, bezogen auf das gesamte zu konservierende Produkt.

**Claims**

1. The use of aminohydroxystearic acid amides corresponding to the following formula

$$CH_3-(CH_2)_a-(\underset{A}{CH}-\underset{B}{CH}-CH_2)_n-(CH_2)_6-CO-X \qquad (I)$$

in which the combinations n=1 and a=7 or n=2 and a=4 or n=3 and a=1 apply and, of the substituents A and B, one represents a hydroxyl group whilst the remaining substituent B or A and the substituent X represent a radical corresponding to the following formula

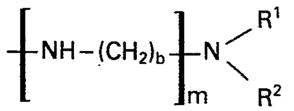 (II)

in which

b represents integers of from 2 to 6,

m = 1 or 2,

$R^1$ and $R^2$ independently of one another represent hydrogen or an alkyl radical containing from 1 to 12 carbon atoms, as antimicrobial agents except for therapeutic purposes.

2. The use of aminohydroxystearic acid amides as claimed in Claim 1 for the production of antimicrobial agents in a quantity of from 0.1 to 10% by weight and preferably in a quantity of from 0.5 to 5% by weight, based on the antimicrobial agent as a whole.

3. The use of aminohydroxystearic acid amides as claimed in Claim 1 as preservatives in a quantity of from 0.1 to 2% by weight, based on the entire product to be preserved.

**Revendications**

1. Utilisation des aminohydroxystéaramides de formule I

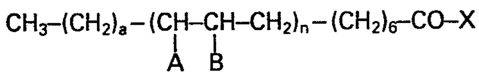 I

dans laquelle les combinaisons n=1 et a=7 ou bien n=2 et a=4 ou bien n=3 et a=1 s'appliquent et, parmi les substituants A et B, l'un représente dans chaque cas un groupe hydroxy cependant que le substituant B ou A restant et le substituant X représentent un reste de formule II

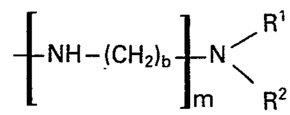 II

dans laquelle b représente un nombre entier de 2 à 6, m est égal à 1 ou 2, $R^1$ et $R^2$ représentent chacun, indépendamment l'un de l'autre, l'hydrogène ou un groupe alkyle en C 1–C 12, en tant que substances actives antimicrobiennes, à l'exception des utilisations thérapeutiques.

2. Utilisation des aminohydroxystéaramides selon la revendication 1 pour la préparation de produits antimicrobiens, en quantités de 0,1 à 10% en poids, de préférence de 0,5 à 5% en poids, par rapport au produit antimicrobien total.

3. Utilisation des aminohydroxystéaramides selon la revendication 1, pour la conservation, en quantitées de 0,1 à 2% en poids, par rapport au produit total à conserver.